# EUROPEAN PATENT SPECIFICATION

(11) **EP 4 452 924 B1**
(45) Date of publication and mention of the grant of the patent: **03.12.2025**
(21) Application number: 22834710.0
(22) Date of filing: 19.12.2022
(51) Int. Cl.: C07C 217/08, C07D 319/08, A61P 11/08

(54) **PROCESS TO PREPARE VILANTEROL TRIFENATATE**
VERFAHREN ZUR HERSTELLUNG VON VILANTEROL-TRIFENATAT
PROCÉDÉ DE PRÉPARATION DU TRIFÉNATATE DE VILANTÉROL

(30) Priority: 22.12.2021 PT 2021117669
(43) Date of publication of application: 30.10.2024
(73) Proprietor: Hovione Scientia Limited, Ringaskiddy, Co. Cork (IE)
(72) Inventor: CARVALHO, Luisa, 2675-409 ODIVELAS (PT); KATZ, Marianna, 1750-014 LISBOA, PORTUGAL (PT); TORRES, Nuno, 1750-014 LISBOA (PT)
(74) Representative: Paulraj, Leonita Theresa
(86) International application number: PCT/GB2022/053293
(87) International publication number: WO 2023/118833

(56) References cited:
- WO-A1-03/024439

## Description

### FIELD OF THE INVENTION

The present invention relates generally to novel processes of preparing vilanterol and pharmaceutically acceptable salts thereof. More specifically the invention pertains to a new process for preparing intermediates for the preparation of vilanterol in batch or continuous flow mode. These new synthetic processes present advantages compared to known routes, including reduction of process time, resources and decreased waste.

### BACKGROUND OF THE INVENTION

Vilanterol trifenatate (**I**) is a long-acting β adrenoreceptor agonist (LABA) with the chemical name (R)-4-(2-((6-(2-((2,6-dichlorobenzyl)oxy)ethoxy)hexyl)amino)-1-hydroxyethyl)-2-(hydroxymethyl)phenol 2,2,2-triphenylacetate [CAS: 503070-58-4] and the following molecular structure:

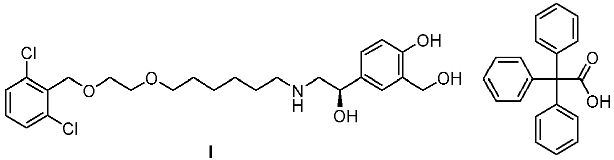

Vilanterol trifenatate (I) was firstly claimed by GlaxoSmithKline in WO 2003/024439. It is approved for use in combination products such as with fluticasone furoate under the tradename Breo Ellipta^{®}; in combination with umeclidinium bromide - Anoro Ellipta^{®} (FDA approval in 2013); and in combination with both - Trelegy Ellipta^{®} (FDA approval in 2017). Combination products are indicated for the treatment of chronic obstructive pulmonary diseases (COPD).

A process to prepare vilanterol and its pharmaceutically acceptable salts, is disclosed in WO 2003/024439, in the corresponding patent application US7361787 and in the J. Med. Chem. 2010, 53, 4522-4530. The reported synthetic sequence includes the preparation of Compound II and Compound III **(Scheme 1).** These key intermediates are coupled in the presence of potassium tert-butoxide to form Compound IV. The oxazolidinone cleavage is performed in THF, in the presence of 4 equivalents of potassium trimethylsilanolate (KOTMS), to afford Compound V. The acetal protecting group is removed in ethanolic acidic conditions, forming vilanterol base (Compound VI). The latter is converted into the corresponding trifenatate salt in ethanol at 80°C.

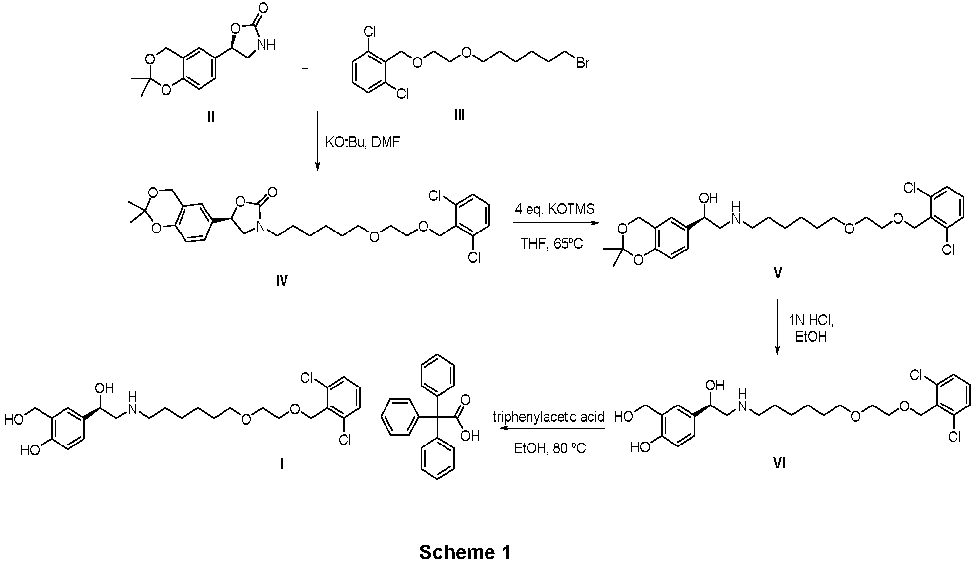

The process described in WO 2003/024439 presents several drawbacks. It requires 4 equivalents of KOTMS under reflux temperature to cleave the oxazolidinone ring. Additionally, it uses an alcoholic solvent for the acetal cleavage that tends to form the corresponding ether impurities. The process requires laborious purifications to purge impurities which are impractical for industrial processes, such as extensive liquid-liquid extractions or chromatographic purifications.

Other companies have claimed to improve the initial synthetic route. Laurus Labs reports in WO 2014/041565 (US 2015/0239862) the preparation of vilanterol trifenatate in alternative alcoholic solvents such as methanol and isopropanol. However, reports that the use of these solvents does not have an exceptional impact on yields or purity. Alternatively, the patent claims that using a non-alcoholic solvent, preferably acetone, results in a decrease in impurity profile (greater than 99.5% purity by HPLC; no single impurity greater than 0.1%). The step comprises the addition of a triphenyl acetic acid slurry to a Compound VI solution, both in acetone, and heating to 50-55°C (example 13, WO 2014/041565). Vilanterol trifenatate is obtained with a purity of 99.79%. In this case the major improvements were performed for the last step, while the overall process drawbacks are maintained.

CN111807973 (Shanghai Anovent Pharmaceutical Co Ltd) discloses a process for the preparation of vilanterol, and relies on the use of succinic acid. WO2017001907 (Teva) also relates to preparing vilanterol, but employs a biocatalytic conversion process involving polypeptides. WO2021033198 (Melody Healthcare PVT Ltd.) employs the use of tetra butyl ammonium hydrogen sulphate (TBAHS) in catalytic amounts in a complicated process.

Israeli patent application 233520 describes a similar synthetic strategy to prepare vilanterol from Compound II and Compound III. In this case, it is disclosed that the use of DMF for coupling step conducts to undesired side-products and it is proposed as alternative solvents, the use of THF or DMSO to prepare Compound IV. The coupling reaction carried out in DMSO requires high temperature (70°C) to attain Compound IV with a purity up to 95 area% by HPLC, while for the reaction carried out in THF, the purity drops to 75.5 area%. Additionally, all the intermediates are still isolated by extraction procedures.

The present inventors, having reviewed these previous reports, have now appreciated that there is still a need to develop more efficient processes to prepare vilanterol and pharmaceutically acceptable salts thereof. As a result of this, the inventors have now devised the present invention, which overcomes, or substantially minimizes, many of the drawbacks found in previous processes.

The inventors found that Compound V and Compound VI, and also the salt (for example compound I) itself, can be efficiently produced via a telescoped approach with improved yields, similar impurity profile while avoiding extensive extraction and isolation steps present verified in the prior art. This approach allows the preparation of vilanterol trifenatate in a one or two-step process which is a relevant improvement when compared to the previous strategies that comprise four synthetic steps. In this way, vilanterol salts may be obtained by a process with an excellent efficiency, saving time, resources and waste. In particular, there is, at most, only isolation of one intermediate (compound V)

### SUMMARY OF THE INVENTION

The present invention relates to novel processes for the preparation of vilanterol and its pharmaceutically acceptable salts thereof, particularly vilanterol trifenatate in batch or continuous flow mode. The invention particularly relates to telescoped processes to prepare vilanterol intermediates, where the starting materials are subjected to successive chemical reactions, without performing work-up and without isolating the intermediates after each reaction. This approach avoids several isolation and purification steps, thus increasing process efficiency and chemical yield.

**Scheme 2** depicts the synthetic sequence that includes telescoped steps to attain compound of Formula I.

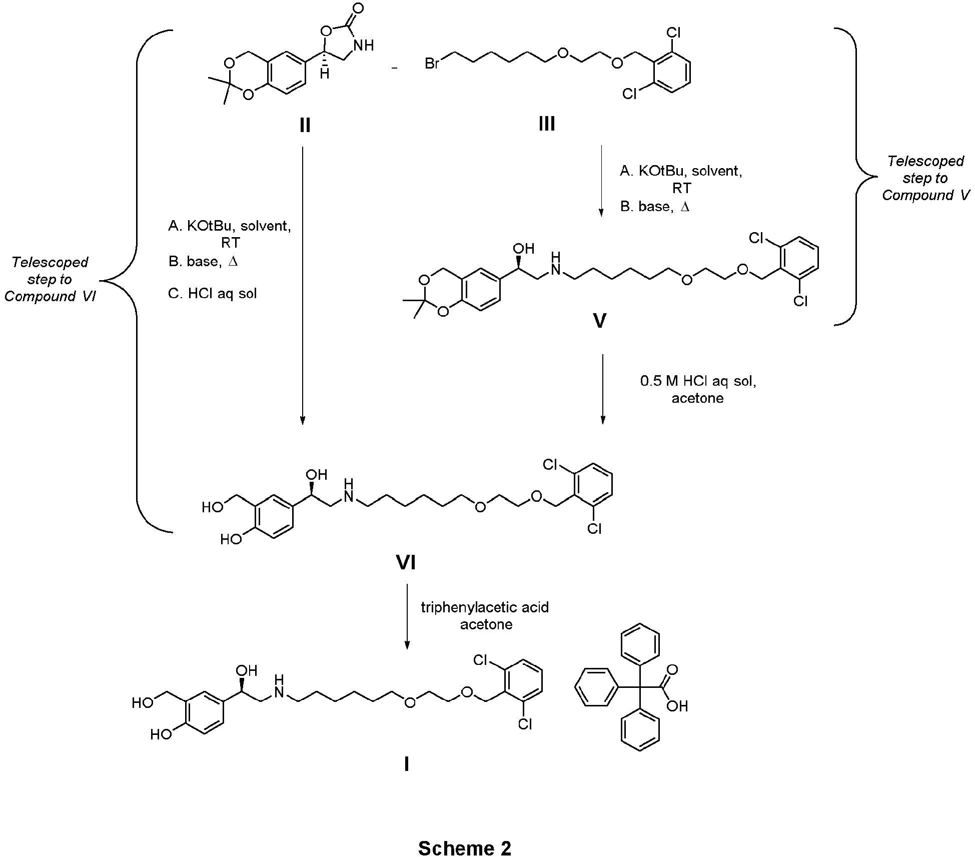

**Scheme 3** depicts a process to attain the Compound of Formula I, wherein no intermediate Compound is isolated.

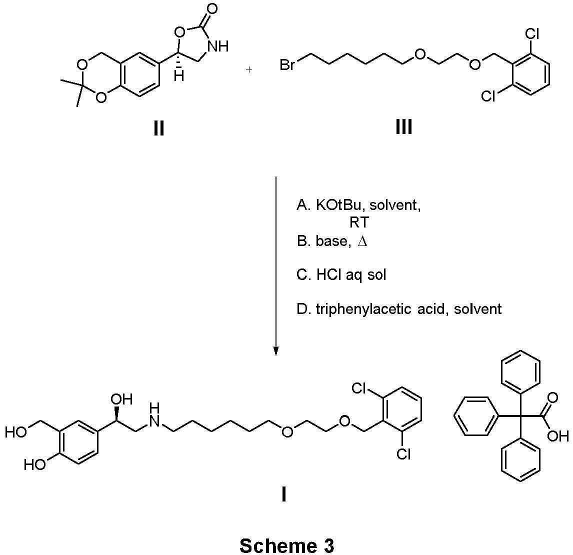

According to one aspect of the present invention, there is provided a process for the preparation of vilanterol ((R)-4-(2-((6-(2-((2,6-dichlorobenzyl)oxy)ethoxy)hexyl)amino)-1-hydroxyethyl)-2-(hydroxymethyl)phenol; Compound VI) or a pharmaceutically acceptable salt thereof, which process comprises the following steps wherein steps A to B(i) are carried out as a one-pot or telescoped process:
A) coupling the Compound (5R)-5-(2,2-dimethyl-4H-1,3-benzodioxin-6-yl)-1,3- oxazolidin-2-one (Compound II) with 2-[2-(6-bromohexyloxy)-ethoxymethyl]-1,3- dichlorobenzene (Compound III) in the presence of a base, in an organic solvent; and
B)
   (i) adding a base to the reaction mixture formed in step A to remove the oxazolidinone protecting group, to form Compound V;
   (ii) converting Compound V to vilanterol (Compound VI) or a pharmaceutically acceptable salt thereof.
As described herein, there is no isolation of intermediate compounds from the reaction mixture (such as Compound IV) until compound V is obtained in step B(i). Compound V may then be isolated if desired, and subsequently converted to vilanterol or a pharmaceutically acceptable salt thereof. Alternatively, as further described below, in one aspect, Compound V is not isolated. The process may be continued as a telescoped or one-pot process, to directly obtain vilanterol or a pharmaceutically acceptable salt thereof, such as vilanterol trifenatate.

The invention thus encompasses a process for the preparation of vilanterol ((R)-4-(2-((6-(2-((2,6-dichlorobenzyl)oxy)ethoxy)hexyl)amino)-1-hydroxyethyl)-2-(hydroxymethyl)phenol; compound VI) or a pharmaceutically acceptable salt thereof, which process comprises coupling the compound (5R)-5-(2,2-dimethyl-4H-1,3-benzodioxin-6-yl)-1,3- oxazolidin-2-one (Compound II) with 2-[2-(6-bromohexyloxy)-ethoxymethyl]-1,3- dichlorobenzene (Compound III) and converting the product formed to vilanterol, wherein only one intermediate compound is isolated. For example, this intermediate compound may be Compound V. The said conversion may, for example, be as per the process steps described above.

According to another aspect of the invention, there is provided a process for the preparation of vilanterol ((R)-4-(2-((6-(2-((2,6-dichlorobenzyl)oxy)ethoxy)hexyl)amino)-1-hydroxyethyl)-2-(hydroxymethyl)phenol; Compound VI) or a pharmaceutically acceptable salt thereof, which process comprises the following steps wherein steps A to C are carried out as a one-pot or telescoped process:
A) coupling the compound (5R)-5-(2,2-dimethyl-4H-1,3-benzodioxin-6-yl)-1,3- oxazolidin-2-one (Compound II) with 2-[2-(6-bromohexyloxy)-ethoxymethyl]-1,3- dichlorobenzene (Compound III) in the presence of a base, in an organic solvent;
B) adding a base to the reaction mixture formed in step A to remove the oxazolidinone protecting group; and
C) adding an acidic solution to the reaction mixture formed in step B to cleave the acetonide protecting group, to form vilanterol (Compound VI);
D) optionally converting Compound VI to a pharmaceutically acceptable salt thereof.
As described herein, in this aspect, there is no isolation of intermediate compounds from the reaction mixture (such as Compound IV, or V) until Compound VI is obtained in step C. Compound VI (vilanterol) may then be isolated if desired, and subsequently converted, if desired, to a pharmaceutically acceptable salt thereof, such as vilanterol trifenatate. Alternatively, as further described below, in one aspect, Compound VI is not isolated. The process may be continued as a telescoped or one-pot process, to directly obtain a pharmaceutically acceptable salt of vilanterol, such as vilanterol trifenatate

The invention thus encompasses a process for the preparation of vilanterol ((R)-4-(2-((6-(2-((2,6-dichlorobenzyl)oxy)ethoxy)hexyl)amino)-1-hydroxyethyl)-2-(hydroxymethyl)phenol; Compound VI) or a pharmaceutically acceptable salt thereof, which process comprises coupling the compound (5R)-5-(2,2-dimethyl-4H-1,3-benzodioxin-6-yl)-1,3- oxazolidin-2-one (Compound II) with 2-[2-(6-bromohexyloxy)-ethoxymethyl]-1,3- dichlorobenzene (Compound III) and converting the product formed to vilanterol, wherein no intermediate compound is isolated. The said conversion may, for example, be as per the process steps described above.

In a further aspect, there is provided a process for the preparation of a pharmaceutically acceptable salt of vilanterol which process comprises the following steps wherein steps A to D are carried out as a one-pot or telescoped process:
A) coupling the compound (5R)-5-(2,2-dimethyl-4H-1,3-benzodioxin-6-yl)-1,3- oxazolidin-2-one (Compound II) with 2-[2-(6-bromohexyloxy)-ethoxymethyl]-1,3- dichlorobenzene (Compound III) in the presence of a base, in an organic solvent;
B) adding a base to the reaction mixture formed in step A to remove the oxazolidinone protecting group; and
C) adding an acidic solution to the reaction mixture formed in step B to cleave the acetonide protecting group, to form vilanterol (Compound VI);
D) converting Compound VI to a pharmaceutically acceptable salt thereof.

As described herein, in this aspect, there is no isolation of intermediate compounds from the reaction mixture (such as Compound IV, V or VI) until a pharmaceutically acceptable salt of vilanterol, such as vilanterol trifenatate, is obtained in step D. The pharmaceutically acceptable salt of vilanterol, such as vilanterol trifenatate, may then be isolated and further purified. In this aspect, the process may thus be operated as a telescoped or one-pot process, to directly obtain a pharmaceutically acceptable salt of vilanterol, such as vilanterol trifenatate

The invention thus encompasses a process for the preparation of a pharmaceutically acceptable salt of vilanterol which process comprises coupling the compound (5R)-5-(2,2-dimethyl-4H-1,3-benzodioxin-6-yl)-1,3- oxazolidin-2-one (Compound II) with 2-[2-(6-bromohexyloxy)-ethoxymethyl]-1,3- dichlorobenzene (Compound III) and converting the product formed to a pharmaceutically acceptable salt of vilanterol, wherein no intermediate compound is isolated. The said conversion may, for example, be as per the process steps described above.

In a preferred aspect of the invention, the pharmaceutically acceptable salt of vilanterol is vilanterol trifenatate.

In one aspect, the present process does not require any purification, particularly it does not require chromatographic purification, for example of intermediate compounds. Thus, the invention includes the feature wherein no purification, particularly no chromatographic purification, is carried out on an intermediate compound produced in the course of the process. Such intermediates, depending on the process used, may include Compound IV, or Compound V, or Compound VI; or both Compounds IV and V , or all of Compounds IV, V and VI.

The same is the case for isolation of an intermediate compound. Thus, the invention includes the feature wherein no isolation is carried out on one or more intermediate compound(s) produced in the course of the process. Such intermediates, depending on the process used, may include Compound IV, or Compound V, or Compound VI; or both Compounds IV and V , or all of Compounds IV, V and VI

In another aspect, the invention provides a process for preparing (R)-3-{6-[2-(2,6-dichlorobenzyloxy)-ethoxy]-hexyl}-5-(2,2-dimethyl-4H-benzo[1,3]dioxin-6-yl)-oxazolidin-2-one 2,2,2-triphenylacetate (Compound I), also known as vilanterol trifenatate, comprising the following steps:
1. A telescoped step comprising:
   A. coupling the compound (5R)-5-(2,2-dimethyl-4H-1,3-benzodioxin-6-yl)-1,3- oxazolidin-2-one (Compound II) with 2-[2-(6-bromohexyloxy)-ethoxymethyl]-1,3- dichlorobenzene (Compound III) in the presence of a base, in a suitable organic solvent;
   B. addition of a base to the reaction mixture to remove the oxazolidinone protecting group; and
   C. addition of an acidic solution to the reaction mixture to remove the acetonide protecting group, to form Compound VI; and
2. Reacting Compound VI with triphenylacetic acid to form Compound I, in a suitable organic solvent.

In another aspect, the invention provides an alternative process for preparing (R)-3-{6-[2-(2,6-dichlorobenzyloxy)-ethoxy]-hexyl}- 5-(2,2-dimethyl-4H-benzo[1,3]dioxin-6-yl)-oxazolidin-2-one 2,2,2-triphenylacetate (Compound I), also known as vilanterol trifenatate, comprising the following steps:
1. A telescoped step comprising:
   A. coupling the compound (5R)-5-(2,2-dimethyl-4H-1,3-benzodioxin-6-yl)-1,3- oxazolidin-2-one (Compound II) with 2-[2-(6-bromohexyloxy)-ethoxymethyl]-1,3- dichlorobenzene (Compound III) in the presence of a base, in a suitable organic solvent; and
   B. addition of a base to the reaction mixture to remove the oxazolidinone protecting group to form Compound V; and
2. Removal of the acetonide protecting group in the presence of an acidic solution in a suitable organic solvent, to produce Compound VI; and
3. Reacting Compound VI with triphenylacetic acid to form Compound I, in a suitable organic solvent.

In another aspect of the present invention, there is provided a process for preparing (R)-4-(2-((6-(2-((2,6-dichlorobenzyl)oxy)ethoxy)hexyl)amino)-1-hydroxyethyl)-2-(hydroxymethyl)phenol (Compound VI), also known as vilanterol, comprising a telescoped step comprising:
A. coupling the compound (5R)-5-(2,2-dimethyl-4H-1,3-benzodioxin-6-yl)-1,3- oxazolidin-2-one (Compound II) with 2-[2-(6-bromohexyloxy)-ethoxymethyl]-1,3- dichlorobenzene (Compound III) in the presence of a base, in a suitable organic solvent;
B. addition of a base to the reaction mixture to remove the oxazolidinone protecting group; and
C. addition of an acidic solution to the reaction mixture for acetonide protecting group cleavage, to form Compound VI.

In a further aspect of the present invention, there is provided a process for preparing (R)-2-((6-(2-((2,6-dichlorobenzyl)oxy)ethoxy)hexyl)amino)-1-(2,2-dimethyl-4H-benzo[d][1,3]dioxin-6-yl)ethan-1-ol (Compound V), comprising a telescoped step comprising:
A. coupling the compound (5R)-5-(2,2-dimethyl-4H-1,3-benzodioxin-6-yl)-1,3- oxazolidin-2-one (Compound II) with 2-[2-(6-bromohexyloxy)-ethoxymethyl]-1,3- dichlorobenzene (Compound III) in the presence of a base, in a suitable organic solvent; and
B. addition of a base to the reaction mixture to remove the oxazolidinone protecting group, to form Compound V.

A one-pot or telescoped process essentially means that the process is carried out without any isolation of intermediate compounds from the reaction mixture. Thus, the usual steps of isolation, and also work-up, or purification of intermediate compounds can be avoided. The final compound (with respect to the process steps in question) can be obtained directly by a series of reactions carried out sequentially or successively, for example in a single vessel or pot, which may for example include a continuous flow reactor or reactors. This approach is also known as telescoping, as will be understood by those in this field. Thus, in one aspect Compound V may be obtained directly from Compounds II and III without isolation of any intermediates, such as Compound IV. Likewise, in other aspects of the present invention, vilanterol itself (Compound VI) or a salt thereof, such as vilanterol trifenatate (Compound I) may be obtained directly from compounds II and III without isolation of any intermediates. The compounds may be obtained in excellent yields, in a more efficient way.

In the present invention, vilanterol or pharmaceutically acceptable salts thereof, such as vilanterol trifenatate, may be obtained using a process having excellent efficiency, thus saving time, resources and waste. In particular, there is, at most, isolation of only one intermediate (Compound V), although in some aspects, as further described, this isolation may also be dispensed with or avoided. The present process has no requirement for chromatographic purification, and it reduces cycle time and process wastes.

The present invention thus describes novel processes to produce vilanterol salts, particularly vilanterol trifenatate. One process comprises an efficient two-step approach that uses as raw materials Compound II and Compound III to form Compound VI via telescoping (i.e. a one pot process) in batch or continuous flow mode. In this telescoped (i.e. a one pot process) batch or continuous flow process the intermediate Compound IV is not isolated, being directly converted into the intermediate Compound V. Subsequently, the intermediate Compound V is not isolated, and it is directly converted into the intermediate Compound VI. The process avoids several isolation and purification steps both in batch and continuous flow modes.

In an alternative process, vilanterol trifenatate is obtained in a three-step approach. In this case, Compound VI is prepared from Compound V which is prepared from Compound II and Compound III via telescoping (i.e. a one-pot process) in batch or continuous flow mode. More specifically, the intermediate Compound IV is not isolated, being directly converted into Compound V. As a final step, vilanterol (Compound VI) may then be converted to a pharmaceutically acceptable salt thereof, suitably vilanterol trifenatate (Compound I).

In an alternative process, we have found that in fact it is also possible to carry out the entire process as a one-pot or telescoped process, effectively in one step, starting from Compounds II and III. Thus, we have found that a pharmaceutically acceptable salt of vilanterol, suitably vilanterol trifenatate (Compound I), can be obtained directly via the process as disclosed herein, without isolation of any of the intermediate compounds.

Compound V and Compound VI and Compound I are obtained via telescoping (i.e. a one-pot process) in batch or continuous flow mode with excellent yields and without the need of chromatographic separation, in all the telescoped (i.e. one-pot) processes described herein.

In one aspect of the invention, the processes described herein do not require the use of succinic acid.

In a further feature, the processes described herein do not employ a biocatalytic conversion process, or the use of polypeptides.

In a further feature, the processes described herein do not employ the use of tetra butyl ammonium hydrogen sulphate (TBAHS).

### DETAILED DESCRIPTION OF THE INVENTION

The present invention relates to novel processes of preparing vilanterol and its pharmaceutically acceptable salts, such as, in particular vilanterol trifenatate. The processes of the invention as described herein may be carried out for example in either batch or continuous flow mode. More specifically, the invention pertains to new processes for preparing Compound V and Compound VI, which are involved in the preparation of vilanterol trifenatate.

In one process, vilanterol (Compound VI) or a pharmaceutically acceptable salt thereof such as vilanterol trifenatate (Compound I) may be prepared, comprising the following steps:
1. A telescoped step, or one-pot process, comprising:
   A. coupling the compound (5R)-5-(2,2-dimethyl-4H-1,3-benzodioxin-6-yl)-1,3- oxazolidin-2-one (Compound II) with 2-[2-(6-bromohexyloxy)-ethoxymethyl]-1,3- dichlorobenzene (Compound III) in the presence of a base, in a suitable organic solvent;
   B. addition of a base to the reaction mixture to remove the oxazolidinone protecting group; and
   C. addition of an acidic solution to the reaction mixture for acetonide protecting group cleavage, to form Compound VI; and
2. reacting Compound VI with triphenylacetic acid to form vilanterol trifenatate (Compound I).

According to the present invention, the process comprises a telescoped step, or one-pot process, to produce Compound VI from Compound II and Compound III, that may be carried out in batch or continuous flow mode.

The reaction (for example, step A), in any aspect of the process, may be carried out in any suitable organic solvent. A polar aprotic solvent is particularly suitable. This may, for example, be selected from the group consisting of dimethyl formamide (DMF), dimethyl sulfoxide (DMSO), dimethyl acetamide (DMAc), n-methyl-pyrrolidin-2-one (NMP), tetrahydrofuran (THF), sulfolane or mixtures thereof; preferably DMSO.

In one particularly preferred aspect, the same solvent may be used for all process steps up to producing Compound V or Compound VI (vilanterol) or a salt thereof. DMSO is preferred for this.

The volume of solvent may for example be at least 10 volumes, or from 10 to 30 volumes, preferably, about 25 volumes, with respect to the volume of Compound II.

According to the present invention, step A comprises coupling Compound II with Compound III in the presence of a base. Compound III may be in excess, preferably up to 1.5 molar equivalents with respect to Compound II, more preferably, Compound III is present in about 1.05 to 1.1 molar equivalents with respect to Compound II. In some aspects, about 1.25 molar equivalents of base may be used with respect to Compound II.

The base may be any base which is suitable for this reaction. For example, the base may be selected from a group consisting of alkali metal hydroxides, alkali metal hydrides such as sodium hydride; alkali metal alkoxides such as sodium *tert*-butoxide, potassium tert-butoxide, preferably potassium tert-butoxide. The base may be used in at least a stoichiometric amount or more, preferably about 1 to about 1.15 molar equivalents of base, or about 1.1 molar equivalents of base, with respect to Compound II. In continuous flow mode the base is preferably used in at least a stoichiometric molar amount or more, with respect to Compound II, preferably about 1.15 to 3 molar equivalents of base with respect to Compound II, or about 1.25 molar equivalents of base with respect to Compound II.

The reaction is preferably carried out at a temperature of from 20 °C to 40 °C, particularly from 20 °C to 30 °C. The reaction may be carried out over a period of 1 to 6 hours, preferably from 1 to 2 hours. In batch mode, step A is preferably carried out at a temperature from 20 to 30°C, preferably from 20 to 25°C.

According to the present invention, upon completion of step A as verified by HPLC, step B comprises the direct addition of a suitable base to the reaction mixture, without carrying out the work-up of the first reaction, to perform the second transformation that is the oxazolidinone ring cleavage. Step B may also comprise the addition of water to the reaction mixture. If used, the water may for example be used in an amount of up to 3 molar equivalents, preferably about 1.5 to 3 molar equivalents with respect to Compound II. The base may be any base that is suitable for carrying out this step, but is preferably selected from a group consisting of sodium or potassium trialkyl silanolates such as potassium trimethylsilanolate; alkali metal alkoxides such as sodium tert-butoxide or potassium tert-butoxide; preferably potassium tert-butoxide. The base is used in an amount up to 3 molar equivalents, preferably about 1.5 to 2 molar equivalents with respect to Compound II. In batch mode, the base used in step B is typically used in an amount of from 1 to 3 molar equivalents with respect to Compound II, preferably about 1.5 molar equivalents with respect to Compound II.

In one aspect of the invention, the process does not employ the use of potassium trimethylsilanolate (KOTMS).

In another feature of the invention, the process may eliminate the use of THF as a solvent, for example in step B of the process.

In another feature of the invention, the process may eliminate the use of acetone as a solvent, for example in step C of the process. We have found this can reduce impurity levels.

The reaction may for example be carried out over a period of 1 to 6 hours, preferably from 1 to 2 hours. The reaction may for example be carried out at a temperature of from 30 to 65°C, preferably from 35 to 50°C. For example, in batch mode, the reaction is typically carried out at a temperature of from 30 to 65°C, preferably from 35 to 50°C.

In some preferred embodiments, the reaction is carried out at about 50°C until reaction completion for up to 6 hours. Preferably, reacting at about 50°C until reaction completion is carried out for 2 hours.

According to the present invention, upon completion of step B as verified by HPLC, step C comprises the direct addition of an acid to the reaction mixture, to perform the third reaction, which is the removal of the acetal protecting group. The acidic solution may be selected from a group consisting of aqueous 0.1 to 1N HCl solutions; alcoholic 1 to 10% HCl solutions; HCl dissolved in suitable organic solvents such as DMF-HCl, ether-HCl, acetic acid-HCl; preferably aqueous HCl solution, for example 1N aqueous HCl solution. The acidic solution may be added to the reaction mixture at a temperature from 0 to 25°C, preferably from 0 to 5°C. The acid addition to the reaction mixture may be performed for up to 4 h, preferably over a period of about 1 to 2h. The reaction may be carried out at a temperature from 20 to 30°C, preferably from 20 to 25°C. The reaction may be carried out over a period of 2 to 10 hours, preferably from 4 to 6 hours. Upon completion of the telescoped step, the reaction mixture may be cooled and neutralized with an aqueous sodium bicarbonate solution, followed by extraction with an organic solvent. Cooling is carried out to temperatures below 20°C, preferably from 10 to 15°C. Extraction is performed with an organic solvent, that may for example be selected from a group consisting of esters and halogenated hydrocarbons, such as ethyl acetate or dichloromethane, preferably ethyl acetate.

According to the present invention, Compound VI obtained by the procedure as described above has a total purity greater than 90%, preferably greater than 95%, as measured by HPLC.

According to the present invention, Compound VI obtained by the procedure as described above, has a chiral purity greater than 99% by HPLC; preferably greater than 99.85%, as measured by HPLC.

According to the present invention, in continuous flow mode step A, step B and step C may comprise reacting a solution of Compound II and a base in a flow reactor to form the deprotonated Compound II; and reacting the deprotonated Compound II and the solution of Compound III in a flow reactor to form Compound IV; and reacting Compound IV and a base in a flow reactor to form Compound V; and reacting Compound V and an acid in a flow reactor or in a batch reactor to form Compound VI. The reaction mixture containing Compound IV may be fed directly to a flow reactor in step B or collected in a buffer tank and fed to a flow reactor for step B. The reaction mixture containing Compound V may be fed directly to a flow reactor for step C, or collected in a buffer tank and fed to a flow reactor for step C.

According to the present invention, step A in continuous flow mode comprises reacting the solution of Compound II and a base in a flow reactor to form the deprotonated Compound II; and reacting the deprotonated Compound II and a solution of Compound III in a flow reactor to form Compound IV. The solution of Compound II and Compound III may be suitably prepared in a polar aprotic solvent that may be selected from the group consisting of dimethyl formamide (DMF), dimethyl sulfoxide (DMSO), dimethyl acetamide (DMAc), n-methyl-pyrrolidin-2-one (NMP), tetrahydrofuran (THF), sulfolane or mixtures thereof; preferably DMSO. The volume of solvent may be at least 10 volumes, preferably about 25 volumes with respect to the volume of Compound II. Compound III may be in excess, preferably it is present in up to 1.5 molar equivalents with respect to Compound II, preferably at about 1.05 molar equivalents. The base may be any suitable base, but is preferably selected from a group consisting of alkali metal hydroxides, alkali metal hydrides such as sodium hydride; alkali metal alkoxides such as sodium tert-butoxide, potassium tert-butoxide, preferably potassium tert-butoxide. The base is suitably used in at least a stoichiometric amount, preferably 1.25 equivalents. In the reaction of Compound II with a base the temperature in the continuous flow reactor is in the range of from 20 °C to 100 °C, preferably around 20 °C. Suitably, the flow rates are adjusted resulting in arrange of from 0.5 min to 30 min residence time, preferably around 2 to 15 minutes, for example about 6 min. In the reaction of the deprotonated Compound II with Compound III the temperature in the continuous flow reactor is preferably in the range of from 20 °C to 100 °C, preferably around 60 °C. Suitably, the flow rates are adjusted resulting in the range of from 0.5 min to 30 min residence time, preferably around 2 to 15 minutes, for example about 6 min. According to the present invention, step B in continuous flow mode suitably comprises reacting the reaction mixture containing Compound IV and a base in a continuous flow reactor. The base may be any base suitable for this step, but is preferably selected from a group consisting of sodium or potassium trialkyl silanolates such as potassium trimethylsilanolate; alkali metal alkoxides such as sodium tert-butoxide or potassium tert-butoxide; preferably potassium tert-butoxide. The base is preferably used in an amount up to 15 molar equivalents with respect to Compound II, preferably about 4 to 5 molar equivalents with respect to compound II. The temperature in the continuous flow reactor is for example in the range of from 20 °C to 150 °C, preferably around 80 °C. The flow rates may be suitably adjusted, typically resulting in the range of from 0.5 min to 30 min residence time, preferably around 2 to 10 minutes, for example about 4 min.

According to the present invention, step C in continuous flow mode comprises reacting the reaction mixture containing Compound V and an acid in a continuous flow reactor or leading the reaction mixture containing Compound V to a batch reactor containing an acid to form Compound VI. The acid may be any acid suitable for performing this step, for example any acidic solution which is suitable to achieve acetal hydrolysis in step C, although is preferably selected from a group consisting of aqueous 0.1 to 1N HCl solutions; alcoholic 1 to 10% HCl solutions; HCl dissolved in suitable organic solvents such as DMF-HCl, ether-HCl, acetic acid-HCl; preferably aqueous HCl solution, such as 1N aqueous HCl solution. The acid may be used in excess, preferably around 1.5 molar equivalents with respect to Compound II.

The temperature in the continuous flow or batch reactor is typically in the range of from 20 °C to 100 °C, preferably around 20 °C. In batch mode, step C is preferably carried out at a temperature between about 20 °C and 30 °C, particularly between about 20 °C and 25 °C. In continuous flow mode, step C is preferably carried out at a temperature in the range of from 20 °C to 100 °C, preferably around 20 °C.

In continuous flow mode in step C, the flow rates are typically adjusted resulting in the range of from 0.5 min to 30 min residence time, preferably around 10 min. In batch mode the reaction may be carried out over a period of 2 to 10 hours, preferably from 4 to 6 hours. The reaction mixture collected in the telescoped batch or continuous flow processes to form Compound VI is cooled and neutralized with an aqueous sodium bicarbonate solution, followed by extraction with an organic solvent. Cooling is carried out to temperatures below 20°C, preferably from 10 to 15°C. Extraction is performed with an organic solvent, which may suitably be selected from a group consisting of esters and halogenated hydrocarbons, such as ethyl acetate or dichloromethane, preferably ethyl acetate.

According to the present invention, Compound VI obtained as in the above process from steps A to C, may be converted into pharmaceutically acceptable salt as a last step of the process, wherein the process comprises reacting Compound VI obtained via telescoping with a suitable acid, preferably triphenyl acetic acid; in the presence of suitable solvent. Use of this acid would provide vilanterol trifenatate as the final product, and this salt is preferred.

According to the present invention, Compound V obtained as in the above process from steps A to B, may be converted into Compound VI that is converted into a pharmaceutically acceptable salt as a last step of the process, wherein the process comprises reacting Compound VI obtained via telescoping with a suitable acid, preferably, triphenyl acetic acid; in the presence of suitable solvent. Use of this acid would provide vilanterol trifenatate as the final product, and this salt is preferred.

According to the present invention, the last step may for example comprise the dissolution of Compound VI in a suitable solvent, and the addition of triphenylacetic acid to form vilanterol trifenatate. The reaction is suitably carried out in a polar aprotic solvent that may for example be selected from the group consisting of methylene chloride (DCM), ethyl acetate, THF, DMF, acetone, acetonitrile or mixtures thereof; preferably acetone.

Also according to the present invention, in a further aspect, as described above, the step of converting vilanterol to a pharmaceutically acceptable salt such as vilanterol trifenatate may also form part of the one-pot process. That is, the telescoped reaction (i.e. one-pot process) may comprise all steps from Step A up until formation of the salt. This may be done either in batch or continuous flow mode.

Thus, if desired, Compound I is obtained in a one-step approach that uses as raw materials Compound II and III via telescoping, for example in batch mode. In this telescoped batch process, intermediate VI is not isolated, being directly converted into Compound I. The process avoids several distillations, isolations and purifications since Compound I can be directly isolated from the reaction mixture by precipitation. In this way, Compound I is prepared in excellent yields and excellent purity. Optionally, recrystallization of Compound I can be performed

In accordance with the invention, Compound I (vilanterol trifenatate) may thus for example be prepared, comprising the following steps:
1. A telescoped batch or continuous flow process comprising:
   A. coupling the compound (5R)-5-(2,2-dimethyl-4H-1,3-benzodioxin-6-yl)-1,3- oxazolidin-2-one (Compound II) with 2-[2-(6-bromohexyloxy)-ethoxymethyl]-1,3- dichlorobenzene (Compound III) in the presence of a base, in a suitable organic solvent;
   B. addition of a base to the reaction mixture to remove the oxazolidinone protecting group to form Compound V; and
2. Removal of the acetonide protecting group in the presence of an acidic solution in a suitable organic solvent, to produce Compound VI; and
3. Reacting Compound VI with triphenylacetic acid to form Compound I.

The invention also encompasses a process of preparing Compound V from Compound II and Compound III, comprising:
A. coupling the compound (5R)-5-(2,2-dimethyl-4H-1,3-benzodioxin-6-yl)-1,3- oxazolidin-2-one (Compound II) with 2-[2-(6-bromohexyloxy)-ethoxymethyl]-1,3- dichlorobenzene (Compound III) in the presence of a base, in a suitable organic solvent; and
B. addition of a base to the reactional mixture to remove the oxazolidinone protecting group to form Compound V.

Compound V may then if desired be isolated and purified, and subsequently converted to Compound VI, and then suitably into a pharmaceutically acceptable salt such as vilanterol trifenatate.

The invention thus also comprises a process of preparing Compound VI from Compound II and Compound III, comprising:
A. coupling the compound (5R)-5-(2,2-dimethyl-4H-1,3-benzodioxin-6-yl)-1,3- oxazolidin-2-one (Compound II) with 2-[2-(6-bromohexyloxy)-ethoxymethyl]-1,3- dichlorobenzene (Compound III) in the presence of a base, in a suitable organic solvent;
B. addition of a base to the reaction mixture to remove the oxazolidinone protecting group; and
C. addition of an acidic solution to the reactional mixture for acetonide protecting group cleavage, to form Compound VI.

The invention thus also comprises a process for the preparation of vilanterol trifenatate (Compound I) according to the process steps described herein, which process comprises, as a final step, reacting Compound VI with triphenylacetic acid to form vilanterol trifenatate (Compound I).

The invention also comprises a process for the preparation of vilanterol trifenatate (Compound I) according to the process steps described herein, which process comprises, as the final steps:
i) Removal of the acetonide protecting group from Compound V in an acidic solution, to form Compound VI; and
ii) Reacting Compound VI with triphenylacetic acid to form Compound I.

The following examples illustrate the present invention but are not intended to be construed as limitations of the present invention.

### EXAMPLES

### Example 1

### Telescoped procedure for the synthesis of (R)-4-(2-((6-(2-((2,6-dichlorobenzyl)oxy)ethoxy)hexyl) amino)-1-hydroxyethyl)-2-(hydroxymethyl)phenol (vilanterol)

Potassium tert-butoxide (1.98 g, 1.1 equiv.) is added to a solution of (5R)-5-(2,2-dimethyl-4H-1,3-benzodioxin-6-yl)-1,3-oxazolidin-2-one (4 g, 16 mmol) in DMSO (15 vol) under N₂ atmosphere and the reaction is stirred for 1 hour at room temperature. A solution of 2-[2-(6-bromo-hexyloxy)-ethoxymethyl]-1,3-dichloro-benzene (6.47 g, 1.1 equiv.) in DMSO (10 vol) is added to the reaction mixture and stirred at room temperature until reaction is completed according to HPLC. Potassium tert-butoxide (2.70 g, 1.5 equiv.) is then added to the reaction mixture and the reaction mixture is heated to 50°C until full conversion according to HPLC. The reaction mixture is cooled and 15% of 1M HCl aqueous solution (4.5 equiv) is added dropwise to the reaction mixture. The reaction mixture is cooled to 0 - 5°C and the remaining 1M HCl aqueous solution is added dropwise. The reaction is stirred at 5°C for 1h and then at room temperature until reaction is completed according to HPLC. The reaction mixture is cooled to 10 - 15°C and neutralized with a 1M NaHCO₃ aqueous solution. The aqueous phase is extracted three times with ethyl acetate (10 vol). The combined organic layer is then washed with water (10 vol). The solvent was distilled and dried under vacuum to afford Compound VI as a brown oil (7.5 g, yield 96%, purity by HPLC: 90.9%).

### Example 2

### Telescoped procedure for the synthesis of (R)-4-(2-((6-(2-((2,6-dichlorobenzyl)oxy)ethoxy)hexyl) amino)-1-hydroxyethyl)-2-(hydroxymethyl)phenol (vilanterol)

Potassium tert-butoxide (1.29 g, 1.15 equiv.) is added to a solution of (5R)-5-(2,2-dimethyl-4H-1,3-benzodioxin-6-yl)-1,3-oxazolidin-2-one (2.5 g, 10 mmol) in DMSO (15 vol) under N₂ atmosphere and the reaction is stirred for 1 hour at room temperature. A solution of 2-[2-(6-bromo-hexyloxy)-ethoxymethyl]-1,3-dichloro-benzene (4.04 g, 1.05 equiv.) in DMSO (10 vol) is added to the reaction mixture and stirred at room temperature until reaction is completed according to HPLC. Potassium tert-butoxide (2.3 g, 2 equiv.) is then added to the reaction mixture and the reaction mixture is heated to 40°C until full conversion according to HPLC. The reaction mixture is cooled to about 15°C and neutralized with a 0.5M HCl aqueous solution. The aqueous phase is extracted three times with ethyl acetate (20 vol). The combined organic layer is then washed with water (60 vol). The solvent was distilled and dried under vacuum to afford Compound VI as a brown oil (5.1 g, yield 96%, purity by HPLC: 82.1%).

### Example 3

### General procedure for the synthesis of vilanterol

A solution of Compound V (8.4 g, 16mmol) in acetone (67 mL) was cooled to 5°C. Then, a 0.5M HCl aqueous solution (80 mL) was added dropwise and the mixture was stirred at this temperature for 1h. The mixture then was stirred at room temperature, until reaction is completed according to HPLC. The reaction mixture was cooled to about 15°C and neutralized with a 1M sodium bicarbonate solution (40 mL). The aqueous phase is extracted three times with ethyl acetate (33 mL). The combined organic layer is then washed with water (67 mL). The solvent was distilled and dried under vacuum to afford Compound VI as a brown oil (4.6 g, yield 59%, purity by HPLC: 82.1%).

### Example 4

### General procedure for the synthesis of vilanterol trifenatate

A slurry of triphenylacetic acid (4.2 g, 1 equiv.) in acetone (14 mL) was added to a solution of 4-((R)-2-{6-[2-(2,6-dichlorobenzyloxy)-ethoxy]-hexylamino}-1-hydroxyethyl-)-2-hydroxy-methyl-phenol (7 g, 14.4 mmol) in acetone (126 mL) and the mixture was heated to 50°C until full dissolution. The mixture was distilled to 12 vol at room temperature, which induced precipitation. The reaction mixture was stirred for up to 20 hours at room temperature. The resulting product was filtered, washed with acetone (14 mL) and isopropyl ether (14 mL), and dried in vacuum at room temperature to afford vilanterol trifenatate as a white crystalline solid (5.6 g, yield 75 w%, purity by HPLC: 96.6%).

### Example 5

### Continuous flow procedure for the synthesis of Compound IV

A solution of (5R)-5-(2,2-dimethyl-4H-1,3-benzodioxin-6-yl)-1,3- oxazolidin-2-one (Compound II) (1 g, 4.01 mmol) in DMSO (10 mL) at 0.012 mL/min flow rate and potassium tert-butoxide (0.56 g, 1.25 equiv.) in DMSO (10 mL) at 0.012 mL/min flow rate were injected into a first PFA coil reactor (0.6 mL) at 20 °C. The resulting mixture and a solution of 2-[2-(6-bromohexyloxy)-ethoxymethyl]-1,3- dichlorobenzene (Compound III) (1.62 g, 1.05 equiv.) in DMSO (10 mL) at 0.012 mL/min flow rate were injected into a second PFA coil reactor. (0.4 mL) at 20 °C. It will be understood the exact amounts and flow rates may be varied as desired. The product was not isolated, the reaction mixture was collected and analyzed by HPLC and showed 97% conversion.

### Example 6

### Continuous flow procedure for the synthesis of Compound IV

A solution of (5R)-5-(2,2-dimethyl-4H-1,3-benzodioxin-6-yl)-1,3- oxazolidin-2-one (Compound II) (1 g, 4.01 mmol) in DMSO (10 mL) at 0.033 mL/min flow rate and potassium tert-butoxide (0.68 g, 1.5 equiv.) in DMSO (10 mL) at 0.033 mL/min flow rate were injected into a PFA coil reactor (0.4 mL) at 20 °C. The resulting mixture and a solution of 2-[2-(6-bromohexyloxy)-ethoxymethyl]-1,3- dichlorobenzene (Compound III) (1.62 g, 1.05 equiv.) in DMSO (10 mL) at 0.033 mL/min flow rate were injected into a PFA coil reactor (0.6 mL) at 60 °C. The product was not isolated, the reaction mixture was collected and analyzed by HPLC and showed 93% conversion.

### Example 7

### Continuous flow procedure for the synthesis of Compound IV

A solution of (5R)-5-(2,2-dimethyl-4H-1,3-benzodioxin-6-yl)-1,3- oxazolidin-2-one (Compound II) (2 g, 8.02 mmol) in DMSO (10 mL) at 0.033 mL/min flow rate and potassium tert-butoxide (1.12 g, 1.25 equiv.) in DMSO (10 mL) at 0.033 mL/min flow rate were injected into a PFA coil reactor (0.3 mL) at 20 °C. The resulting mixture and a solution of 2-[2-(6-bromohexyloxy)-ethoxymethyl]-1,3- dichlorobenzene (Compound III) (3.08 g, 1.0 equiv.) in DMSO (10 mL) at 0.033 mL/min flow rate were injected into a PFA coil reactor (0.6 mL) at 100 °C. The product was not isolated, the reaction mixture was collected and analyzed by HPLC and showed 98.5% conversion. The molar equivalent amounts used in this example may give rise to lower impurities.

### Example 8

### Continuous flow procedure for the synthesis of Compound V

A solution of Compound IV (previously isolated from batch trials) (2.22 g, 4.01 mmol) in DMSO (25 mL) at 0.05 mL/min flow rate and potassium tert-butoxide (0.4 g, 2.2 equiv.) in DMSO (10 mL) at 0.05 mL/min flow rate were injected into a PFA coil reactor (0.6 mL) at 80 °C. The product was not isolated, the reaction mixture was collected and analyzed by HPLC and showed 90% conversion.

### Example 9

### Continuous flow procedure for the synthesis of Compound V

A solution of Compound IV (previously isolated from batch trials (2.22 g, 4.01 mmol) in DMSO (25 mL) at 0.05 mL/min flow rate and potassium tert-butoxide (0.4 g, .4.4 equiv.) in DMSO (10 mL) at 0.1 mL/min flow rate were injected into a PFA coil reactor (0.6 mL) at 80 °C. The product was not isolated, the reaction mixture was collected and analyzed by HPLC and showed 99.9% conversion.

### Example 10

### Continuous flow procedure for the synthesis of Compound VI

A solution of Compound V (previously isolated from batch trials (1 g, 1.81 mmol) in DMSO (8 mL) at 0.15 mL/min flow rate and 1M HCl aqueous solution (4.3 equiv.) at 0.15 mL/min flow rate were injected into a PFA coil reactor (0.3 mL) at 20 °C. The product was not isolated, the reaction mixture was collected and analyzed by HPLC resulting in 27% conversion.

### Example 11

### Continuous flow procedure for the synthesis of Compound VI

A solution of Compound V (1 g, 1.81 mmol) in DMSO (8 mL) at 0.025 mL/min flow rate and 1M HCl aqueous solution (4.3 equiv.) at 0.025 mL/min flow rate were injected into a PFA coil reactor (0.3 mL) at 20 °C. The product was not isolated, the reaction mixture was collected and analyzed by HPLC and showed 87% conversion. The flow rates used in this example may account for the higher conversion.

### Example 12

### Telescoped continuous flow procedure for the synthesis of Compound V

A solution of (5R)-5-(2,2-dimethyl-4H-1,3-benzodioxin-6-yl)-1,3-oxazolidin-2-one (Compound II) (1 g, 4.01 mmol) in DMSO (10 mL) at 0.033 mL/min flow rate and potassium tert-butoxide (0.68 g, 1.5 equiv.) in DMSO (10 mL) at 0.033 mL/min flow rate were injected into a PFA coil reactor (0.4 mL) at 20 °C. The resulting mixture and a solution of 2-[2-(8-bromohexyloxy)-ethoxymethyl]-1,3-dichlorobenzene (Compound III) (1.62 g, 1.05 equiv.) in DMSO (10 mL) at 0.033 mL/min flow rate were injected into a PFA coil reactor (0.6 mL) at 20 °C. The resulting mixture and potassium tert-butoxide (0.66 g, 13 equiv.) in DMSO (10 mL) at 0.1 mL/min flow rate were injected into a PFA coil reactor (0.6 mL) at 80 °C. The product was not isolated, the reaction mixture was collected and analyzed by HPLC and showed 89.5% conversion.

### Example 13

### Telescoped continuous flow procedure for the synthesis of Compound VI

A solution of (5R)-5-(2,2-dimethyl-4H-1,3-benzodioxin-6-yl)-1,3-oxazolidin-2-one (Compound II) (1 g, 4.01 mmol) in DMSO (10 mL) at 0.033 mL/min flow rate and potassium tert-butoxide (0.68 g, 1.5 equiv.) in DMSO (10 mL) at 0.033 mL/min flow rate were injected into a PFA coil reactor (0.4 mL) at 20 °C. The resulting mixture and a solution of 2-[2-(8-bromohexyloxy)-ethoxymethyl]-1,3-dichlorobenzene (Compound III) (1.62 g, 1.05 equiv.) in DMSO (10 mL) at 0.033 mL/min flow rate were injected into a PFA coil reactor (0.6 mL) at 20 °C. The resulting mixture and potassium tert-butoxide (0.66 g, 13 equiv.) in DMSO (10 mL) at 0.1 mL/min flow rate were injected into a PFA coil reactor (0.6 mL) at 80 °C. The resulting mixture and 1M HCl aqueous solution at 0.2 mL/min flow rate were injected into a PFA coil reactor (2.4 mL) at 20 °C. The product was not isolated, the reaction mixture was collected and analyzed by HPLC and showed 85% conversion.

### Example 14

### Telescoped continuous flow procedure for the synthesis of Compound VI

A solution of (5R)-5-(2,2-dimethyl-4H-1,3-benzodioxin-6-yl)-1,3-oxazolidin-2-one (Compound II) (1 g, 4.01 mmol) in DMSO (10 mL) at 0.033 mL/min flow rate and potassium tert-butoxide (0.68 g, 1.5 equiv.) in DMSO (10 mL) at 0.033 mL/min flow rate were injected into a PFA coil reactor (0.4 mL) at 20 °C. The resulting mixture and a solution of 2-[2-(8-bromohexyloxy)-ethoxymethyl]-1,3-dichlorobenzene (Compound III) (1.62 g, 1.05 equiv.) in DMSO (10 mL) at 0.033 mL/min flow rate were injected into a PFA coil reactor (0.6 mL) at 20 °C. The resulting mixture and potassium tert-butoxide (0.66 g, 13 equiv.) in DMSO (10 mL) at 0.1 mL/min flow rate were injected into a PFA coil reactor (0.6 mL) at 80 °C. The resulting mixture was cooled to about 15°C and neutralized with a 1M HCl aqueous solution. The product was not isolated, the reaction mixture was collected and analyzed by HPLC and showed 88% conversion.

### Preparation of vilanterol pharmaceutically acceptable salts.

### Example 15:

Potassium tert-butoxide (9.4 g, 1.1 equiv.) is added to DMSO (15 vol.) under N2 atmosphere and the reaction is stirred for 1 hour at room temperature. (5R)-5-(2,2-dimethyl-4H-1,3- benzodioxin-6-yl)-1,3-oxazolidin-2-one (19.0 g, 76.1 mmol) is added and the reaction is stirred for 1 hour at room temperature. A solution of 2-[2-(6-bromo-hexyloxy)-ethoxymethyl]-1,3- dichloro-benzene (32.6 g, 1.09 equiv.) in DMSO (5 vol) is added to the reaction mixture and stirred at room temperature until reaction is completed according to HPLC. Water (2.1 mL, 1.5 eq.) and Potassium tert-butoxide (18.0 g, 2.1 equiv.) is then added to the reaction mixture and the reaction mixture is heated to 35°C until full conversion according to HPLC. The reaction mixture is cooled to 10°C and 16% of 0.5M HCl aqueous solution (3.5 equiv.) is added dropwise to the reaction mixture. The reaction mixture is stirred at 25°C and the remaining 0.5M HCl aqueous solution is added dropwise. The reaction is stirred at 25°C until reaction is completed according to HPLC. The aqueous phase is extracted with ethyl acetate (19.5 vol). The reaction mixture is cooled to 10 - 15°C and neutralized with a 1M Na₂CO₃ aqueous solution. The aqueous phase is extracted two times with ethyl acetate (10 vol). The combined organic layer is then washed with water (10 vol). The organic layer is added to a mixture of EtOAc (14 vol.) followed by triphenylacetic acid addition (22 g, 1.0 equiv) stirred for 1 hour at room temperature. The reaction is cooled down to 0°C and stirred for 6 hours. The solid is isolated by filtration and washed twice with EtOAc (8 vol.). The solid is dried under vacuum to afford Compound I as an off-white solid (37.8 g, yield 64%, purity by HPLC: 99.2%).

### Example 16:

Potassium tert-butoxide (14.9 g, 1.1 equiv.) is added to DMSO (15 vol.) under N2 atmosphere and the reaction is stirred for 1 hour at room temperature. (5R)-5-(2,2-dimethyl-4H-1,3- benzodioxin-6-yl)-1,3-oxazolidin-2-one (3.5 g, 14.1 mmol) is added and the reaction is stirred for 1 hour at room temperature. A solution of 2-[2-(6-bromo-hexyloxy)-ethoxymethyl]-1,3- dichloro-benzene (6.0 g, 1.09 equiv.) in DMSO (5 vol) is added to the reaction mixture and stirred at room temperature until reaction is completed according to HPLC. Water (0.76 mL, 3.0 eq.) and Potassium tert-butoxide (2.5 g, 2.1 equiv.) is then added to the reaction mixture and the reaction mixture is heated to 35°C until full conversion according to HPLC. Water (3.5 mL) was added and the reaction mixture is cooled to 5°C. 0.5M HCl aqueous solution (3.5 equiv.) is added dropwise to the reaction mixture. The reaction is stirred at 25°C until reaction is completed according to HPLC. The aqueous phase is extracted with ethyl acetate (19.5 vol). The reaction mixture is cooled to 10 - 15°C and neutralized with a 1M Na₂CO₃ aqueous solution. The aqueous phase is extracted two times with ethyl acetate (10 vol). The combined organic layer is then washed with water (10 vol). The organic layer is added to EtOAc (22 vol.) followed by triphenylacetic acid addition (4.1 g, 1.0 equiv) stirred for 1 hour at room temperature. The reaction is cool down to 0°C and stirred for 6 hours. The solid is isolated by filtration and washed twice with EtOAc (8 vol.). The solid is dried under vacuum to afford Compound I as an off-white solid (3.5g, yield 32%, purity by HPLC: 99.4%).

### Example 17:

Potassium tert-butoxide (4.21 g, 1.1 equiv.) is added to DMSO (15 vol.) under N2 atmosphere and the reaction is stirred for 1 hour at room temperature. (5R)-5-(2,2-dimethyl-4H-1,3- benzodioxin-6-yl)-1,3-oxazolidin-2-one (8.5 g, 34 mmol) is added and the reaction is stirred for 1 hour at room temperature. A solution of 2-[2-(6-bromo-hexyloxy)-ethoxymethyl]-1,3- dichloro-benzene (14.63 g, 1.09 equiv.) in DMSO (5 vol) is added to the reaction mixture and stirred at room temperature until reaction is completed according to HPLC. Water (0.95 mL, 1.5 eq.) and Potassium tert-butoxide (8.0 g, 2.1 equiv.) is then added to the reaction mixture and the reaction mixture is heated to 35°C until full conversion according to HPLC. Water (8.5 mL) was added and the reaction mixture is cooled to 5°C. 0.5M HCl aqueous solution (3.5 equiv.) is added dropwise to the reaction mixture. The reaction is stirred at 25°C until reaction is completed according to HPLC. The aqueous phase is extracted with ethyl acetate (19.5 vol). The reaction mixture is cooled to 10 - 15°C and neutralized with a 1M Na₂CO₃ aqueous solution. The aqueous phase is extracted two times with ethyl acetate (10 vol). The combined organic layer is then washed with water (10 vol). The organic layer is divided in two equal parts (A and B).

Part A is added to EtOAc (22 vol.) followed by triphenylacetic acid addition (4.9 g, 1.0 equiv) stirred for 1 hour at room temperature. The reaction is cool down to 0°C and stirred for 6 hours. The solid is isolated by filtration and washed twice with EtOAc (4 vol.). The solid is dried under vacuum to afford Compound I as an off-white solid (6.1g, yield 46.1%, purity by HPLC: 99.2%).

Part B is added to mixture of EtOAc: water 2.8% (22 vol.) followed by triphenylacetic acid addition (4.9 g, 1.0 equiv) stirred for 1 hour at room temperature. The reaction is cool down to 0°C and stirred for 6 hours. The solid is isolated by filtration and washed twice with EtOAc: water 2.8% (4 vol.). The solid is dried under vacuum to afford Compound I as an off-white solid (7.2g, yield 54.6 %, purity by HPLC: 99.4%).

### Example 18:

### Recrystallization of vilanterol trifenatate

Compound I (6.6 g, 7.2 mmol) is added to mixture of EtOAc: water 2.8% (50 vol.). The suspension is heat up to 45°C and stirred for 1 hour with dissolution of the compound I. The solution is cooled down to 0°C and stirred for 6 hours. The solid is isolated by filtration and washed twice with EtOAc: water 2.8% (4 vol.). The solid is dried under vacuum to afford Compound I as an off-white solid (4.4g, yield 67.5 %, purity by HPLC 99.6%).

### Example 19

### Recrystallization of vilanterol trifenatate

Compound I (5.6g, 7.2 mmol) is added to EtOAc (55 vol.) The suspension is heat up to 60°C and stirred for 1 hour with dissolution of the compound I. The solution is cooled down to 10°C and stirred for 6 hours. The solid is isolated by filtration and washed twice with EtOAc (4 vol.). The solid is dried under vacuum to afford Compound I as an off-white solid (4.52g, yield 81.0 %, purity by HPLC 99.5%).

## Claims

1. A process for the preparation of vilanterol ((R)-4-(2-((6-(2-((2,6-dichlorobenzyl)oxy)ethoxy)hexyl)amino)-1-hydroxyethyl)-2-(hydroxymethyl)phenol; Compound VI) or a pharmaceutically acceptable salt thereof, which process comprises the following steps wherein steps A to B (i) are carried out as a one-pot process:
A) coupling the compound (5R)-5-(2,2-dimethyl-4H-1,3-benzodioxin-6-yl)-1,3- oxazolidin-2-one (Compound II) with 2-[2-(6-bromohexyloxy)-ethoxymethyl]-1,3-dichlorobenzene (Compound III) in the presence of a base, in an organic solvent; and
B)
(i) adding a base to the reaction mixture formed in step A in the presence of said organic solvent to remove the oxazolidinone protecting group, to form Compound V;
(ii) converting compound V to vilanterol (Compound VI) or a pharmaceutically acceptable salt thereof.

2. A process according to claim 1 for the preparation of vilanterol ((R)-4-(2-((6-(2-((2,6-dichlorobenzyl)oxy)ethoxy)hexyl)amino)-1-hydroxyethyl)-2-(hydroxymethyl)phenol; Compound VI) or a pharmaceutically acceptable salt thereof, which process comprises the following steps wherein steps A to C are carried out as a one-pot process:
A) coupling the compound (5R)-5-(2,2-dimethyl-4H-1,3-benzodioxin-6-yl)-1,3- oxazolidin-2-one (Compound II) with 2-[2-(6-bromohexyloxy)-ethoxymethyl]-1,3-dichlorobenzene (Compound III) in the presence of a base, in an organic solvent;
B) adding a base to the reaction mixture formed in step A in the presence of said organic solvent to remove the oxazolidinone protecting group; and
C) adding an acidic solution to the reaction mixture formed in step B in the presence of said organic solvent to cleave the acetonide protecting group, to form vilanterol (Compound VI);
D) optionally converting Compound VI to a pharmaceutically acceptable salt thereof.

3. A process according to claim 1 or 2 for the preparation of a pharmaceutically acceptable salt of vilanterol which process comprises the following steps wherein steps A to D are carried out as a one-pot process:
A) coupling the compound (5R)-5-(2,2-dimethyl-4H-1,3-benzodioxin-6-yl)-1,3- oxazolidin-2-one (Compound II) with 2-[2-(6-bromohexyloxy)-ethoxymethyl]-1,3-dichlorobenzene (Compound III) in the presence of a base, in an organic solvent;
B) adding a base to the reaction mixture formed in step A in the presence of said organic solvent to remove the oxazolidinone protecting group; and
C) adding an acidic solution to the reaction mixture formed in step B in the presence of said organic solvent to cleave the acetonide protecting group, to form vilanterol (Compound VI);
D) converting Compound VI to a pharmaceutically acceptable salt thereof.

4. A process according to any preceding claim further comprises at least one the following features:
- wherein there is no isolation of intermediates until Compound V is obtained;
- wherein there is no isolation of intermediates until Compound VI is obtained; and
- wherein there is no isolation of intermediates until a pharmaceutically acceptable salt of vilanterol (Compound VI) is obtained in step D.

5. A process according to any preceding claim, wherein the process is carried out in batch mode or continuous flow mode.

6. A process according to any preceding claim further comprises at least one of the following features:
- wherein the organic solvent in step A is selected from the group consisting of polar aprotic solvent, such as dimethyl formamide, dimethyl sulfoxide, dimethyl acetamide, n-methyl-pyrrolidin-2-one, ethers such as tetrahydrofuran, sulfolane or mixtures thereof; preferably DMSO;
- wherein the volume of solvent with respect to Compound II is at least 10 volumes, preferably 25 volumes;
- wherein the base used in step A is selected from the group consisting of alkali metal hydroxides, alkali metal hydrides or alkali metal alkoxides, preferably potassium tert-butoxide; and
- wherein in batch mode step A is carried out at a temperature from 20 to 30°C, preferably from 20 to 25°C.

7. A process according to any preceding claim wherein in batch mode the base is used in at least a stoichiometric molar amount or more, with respect to Compound II, preferably about 1 to about 1.15 molar equivalents of base, is used with respect to Compound II.

8. A process according to any one of claims 1 to 6 wherein in continuous flow mode the base is used in at least a stoichiometric molar amount or more, with respect to Compound II, preferably about 1.15 to 3 molar equivalents of base is used with respect to Compound II.

9. A process according to any one of claims 1 to 6, or 8 further comprises at least one of the following features:
- wherein in continuous flow mode the reaction of Compound II with a base in step A is carried out at a temperature in the range of from 20 °C to 100 °C, preferably around 20 °C, and the flow rates are adjusted resulting in the range of from 0.5 min to 30 min residence time, preferably around 6 min; and
- wherein in continuous flow mode in the reaction of deprotonated Compound II with Compound Ill in step A is carried out at a temperature in the range of from 20 °C to 100 °C, preferably around 60 °C, and the flow rates are adjusted resulting in the range of from 0.5 min to 30 min residence time, preferably around 6 min.

10. A process according to any one of claims 1 to 7 further comprises at least one of the following features:
- wherein in batch mode the base used in step B is used in an amount of from 1 to 3 molar equivalents with respect to Compound II, preferably 1.5 molar equivalents with respect to Compound II, preferably the base is water; and
- wherein in batch mode the reaction in step B is carried out at a temperature from 30 to 65°C, preferably from 35 to 50°C.

11. A process according to claim any one of claims 1 to 6, 8 -9, further comprises at least one of the following features:
- wherein in continuous flow mode the base used in step B is used in an amount up to 15 molar equivalents with respect to Compound II, preferably about 4 to 5 molar equivalents with respect to compound II;
- wherein in continuous flow mode the reaction in step B is carried out at a temperature in the range of from 20 °C to 150 °C, preferably around 80 °C; and
- wherein in continuous flow mode in step B the flow rates are adjusted resulting in the range of from 0.5 min to 30 min residence time, preferably around 4 min.

12. A process according to any one of claims 2 to 11 wherein the acidic solution used in step C is selected from the group consisting of 0.1 to 1N HCl solutions; alcoholic 1 to 10% HCl solutions; HCl dissolved in suitable organic solvents such as DMF-HCl, ether-HCl, acetic acid-HCl; preferably 1N aqueous HCl solution, or is any acidic solution which is suitable to achieve acetal hydrolysis in step C.

13. A process according to any one of claims 1 to 12 further comprises at least one of the following features:
- wherein in batch mode step C is carried out at a temperature of from about 20 °C and 30 °C, particularly of from about 20 °C and 25 °C;
-- wherein in continuous flow mode step C is carried out at a temperature in the range of from 20 °C to 100 °C, preferably around 20 °C; and
- wherein in continuous flow mode in step C the flow rates are adjusted resulting in the range of from 0.5 min to 30 min residence time, preferably around 10 min.

14. A process for the preparation of vilanterol trifenatate (Compound I) according to any preceding claim which process comprises reacting compound VI with triphenylacetic acid to form vilanterol trifenatate (Compound I).

15. A process for the preparation of vilanterol trifenatate (Compound I) according to any one of claims 1, or 4, or 5 to 14 which process comprises:
i) Removal of the acetonide protecting group from Compound V in an acidic solution, to form Compound VI; and
ii) Reacting compound VI with triphenylacetic acid to form Compound I.

## Patentansprüche

1. Verfahren zur Herstellung von Vilanterol ((R)-4-(2-((6-(2-((2,6-Dichlorbenzyl)oxy)ethoxy)hexyl)amino)-1-hydroxyethyl)-2-(hydroxymethyl)phenol; Verbindung VI) oder eines pharmazeutisch verträglichen Salzes davon, wobei das Verfahren die folgenden Schritte umfasst, wobei Schritt A bis B (i) als Eintopfverfahren durchgeführt werden:
A) Koppeln der Verbindung (5R)-5-(2,2-Dimethyl-4H-1,3-benzodioxin-6-yl)-1,3-oxazolidin-2-on (Verbindung II) mit 2-[2-(6-Bromhexyloxy)-ethoxymethyl]-1,3-dichlorbenzol (Verbindung III) in der Gegenwart einer Base, in einem organischen Lösungsmittel; und
B)
(i) Hinzufügen einer Base zu dem Reaktionsgemisch, das in Schritt A gebildet wird, in der Gegenwart des organischen Lösungsmittels, um die Oxazolidinon-Schutzgruppe zu entfernen, um Verbindung V zu bilden;
(ii) Umwandeln von Verbindung V in Vilanterol (Verbindung VI) oder ein pharmazeutisch verträgliches Salz davon.

2. Verfahren nach Anspruch 1 zur Herstellung von Vilanterol ((R)-4-(2-((6-(2-((2,6-Dichlorbenzyl)oxy)ethoxy)hexyl)amino)-1-hydroxyethyl)-2-(hydroxymethyl)phenol; Verbindung VI) oder eines pharmazeutisch verträglichen Salzes davon, wobei das Verfahren die folgenden Schritte umfasst, wobei Schritt A bis C als Eintopfverfahren durchgeführt werden:
A) Koppeln der Verbindung (5R)-5-(2,2-Dimethyl-4H-1,3-benzodioxin-6-yl)-1,3-oxazolidin-2-on (Verbindung II) mit 2-[2-(6-Bromhexyloxy)-ethoxymethyl]-1,3-dichlorbenzol (Verbindung III) in der Gegenwart einer Base, in einem organischen Lösungsmittel;
B) Hinzufügen einer Base zu dem Reaktionsgemisch, das in Schritt A gebildet wird, in der Gegenwart des organischen Lösungsmittels, um die Oxazolidinon-Schutzgruppe zu entfernen; und
C) Hinzufügen einer sauren Lösung zu dem Reaktionsgemisch, das in Schritt B gebildet wird, in der Gegenwart des organischen Lösungsmittels, um die Acetonid-Schutzgruppe zu spalten, um Vilanterol (Verbindung VI) zu bilden;
D) optional Umwandeln von Verbindung VI in ein pharmazeutisch verträgliches Salz davon.

3. Verfahren nach Anspruch 1 oder 2 zur Herstellung eines pharmazeutisch verträglichen Salzes von Vilanterol, wobei das Verfahren die folgenden Schritte umfasst, wobei Schritt A bis D als Eintopfverfahren durchgeführt werden:
A) Koppeln der Verbindung (5R)-5-(2,2-Dimethyl-4H-1,3-benzodioxin-6-yl)-1,3-oxazolidin-2-on (Verbindung II) mit 2-[2-(6-Bromhexyloxy)-ethoxymethyl]-1,3-dichlorbenzol (Verbindung III) in der Gegenwart einer Base, in einem organischen Lösungsmittel;
B) Hinzufügen einer Base zu dem Reaktionsgemisch, das in Schritt A gebildet wird, in der Gegenwart des organischen Lösungsmittels, um die Oxazolidinon-Schutzgruppe zu entfernen; und
C) Hinzufügen einer sauren Lösung zu dem Reaktionsgemisch, das in Schritt B gebildet wird, in der Gegenwart des organischen Lösungsmittels, um die Acetonid-Schutzgruppe zu spalten, um Vilanterol (Verbindung VI) zu bilden;
D) Umwandeln von Verbindung VI in ein pharmazeutisch verträgliches Salz davon.

4. Verfahren nach einem vorhergehenden Anspruch, ferner umfassend zumindest eines der folgenden Merkmale:
- wobei es keine Isolierung von Zwischenprodukten gibt, bis Verbindung V erhalten wird;
- wobei es keine Isolierung von Zwischenprodukten gibt, bis Verbindung VI erhalten wird; und
- wobei es keine Isolierung von Zwischenprodukten gibt, bis ein pharmazeutisch verträgliches Salz von Vilanterol (Verbindung VI) in Schritt D erhalten wird.

5. Verfahren nach einem vorhergehenden Anspruch, wobei das Verfahren in Batch-Modus oder kontinuierlichem Flussmodus durchgeführt wird.

6. Verfahren nach einem vorhergehenden Anspruch, ferner umfassend zumindest eines der folgenden Merkmale:
- wobei das organische Lösungsmittel in Schritt A ausgewählt ist aus der Gruppe bestehend aus polarem aprotischen Lösungsmittel, wie Dimethylformamid, Dimethylsulfoxid, Dimethylacetamid, n-Methyl-pyrrolidin-2-on, Ethern wie Tetrahydrofuran, Sulfolan oder Gemischen davon; bevorzugt DMSO;
- wobei das Volumen von Lösungsmittel in Bezug auf Verbindung II zumindest 10 Volumen, bevorzugt 25 Volumen ist;
- wobei die Base, die in Schritt A verwendet wird, ausgewählt ist aus der Gruppe bestehend aus Alkalimetallhydroxiden, Alkalimetallhydriden oder Alkalimetallalkoxiden, bevorzugt Kalium-tert-butoxid; und
- wobei in Batch-Modus Schritt A bei einer Temperatur von 20 bis 30 °C, bevorzugt von 20 bis 25 °C durchgeführt wird.

7. Verfahren nach einem vorhergehenden Anspruch, wobei in Batch-Modus die Base in zumindest einer stöchiometrischen molaren Menge oder mehr in Bezug auf Verbindung II verwendet wird, bevorzugt etwa 1 bis etwa 1,15 molare Äquivalente an Base in Bezug auf Verbindung II verwendet werden.

8. Verfahren nach einem der Ansprüche 1 bis 6, wobei in kontinuierlichem Flussmodus die Base in zumindest einer stöchiometrischen molaren Menge oder mehr in Bezug auf Verbindung II verwendet wird, bevorzugt etwa 1,15 bis 3 molare Äquivalente an Base in Bezug auf Verbindung II verwendet werden.

9. Verfahren nach einem der Ansprüche 1 bis 6 oder 8, ferner umfassend zumindest eines der folgenden Merkmale:
- wobei in kontinuierlichem Flussmodus die Reaktion von Verbindung II mit einer Base in Schritt A bei einer Temperatur in dem Bereich von 20 °C bis 100 °C, bevorzugt um 20 °C durchgeführt wird und die Flussraten eingestellt werden, was in dem Bereich von 0,5 min bis 30 min Verweilzeit, bevorzugt um 6 min, resultiert; und
- wobei in kontinuierlichem Flussmodus in der Reaktion von deprotonierter Verbindung II mit Verbindung III in Schritt A bei einer Temperatur in dem Bereich von 20 °C bis 100 °C, bevorzugt um 60 °C, durchgeführt wird und die Flussraten eingestellt werden, was in dem Bereich von 0,5 min bis 30 min Verweilzeit, bevorzugt um 6 min, resultiert.

10. Verfahren nach einem der Ansprüche 1 bis 7, ferner umfassend zumindest eines der folgenden Merkmale:
- wobei in Batch-Modus die Base, die in Schritt B verwendet wird, in einer Menge von 1 bis 3 molaren Äquivalenten in Bezug auf Verbindung II, bevorzugt 1,5 molaren Äquivalenten in Bezug auf Verbindung II verwendet wird, bevorzugt die Base Wasser ist; und
- wobei in Batch-Modus die Reaktion in Schritt B bei einer Temperatur von 30 bis 65 °C, bevorzugt von 35 bis 50 °C durchgeführt wird.

11. Verfahren nach einem der Ansprüche 1 bis 6, 8-9, ferner umfassend zumindest eines der folgenden Merkmale:
- wobei in kontinuierlichem Flussmodus die Base, die in Schritt B verwendet wird, in einer Menge von bis zu 15 molaren Äquivalenten in Bezug auf Verbindung II, bevorzugt etwa 4 bis 5 molaren Äquivalenten in Bezug auf Verbindung II verwendet wird;
- wobei in kontinuierlichem Flussmodus die Reaktion in Schritt B bei einer Temperatur in dem Bereich von 20 °C bis 150 °C, bevorzugt um 80 °C durchgeführt wird; und
- wobei in kontinuierlichem Flussmodus in Schritt B die Flussraten eingestellt werden, was in dem Bereich von 0,5 min bis 30 min Verweilzeit, bevorzugt um 4 min, resultiert.

12. Verfahren nach einem der Ansprüche 2 bis 11, wobei die saure Lösung, die in Schritt C verwendet wird, ausgewählt ist aus der Gruppe bestehend aus 0,1 bis 1N HCl-Lösungen; alkoholischen 1 bis 10 % HCl-Lösungen; HCl gelöst in geeigneten organischen Lösungsmitteln wie DMF-HCl, Ether-HCl, Essigsäure-HCl; bevorzugt 1N wässriger HCl-Lösung, oder eine beliebige saure Lösung ist, die dazu geeignet ist, Acetalhydrolyse in Schritt C zu erreichen.

13. Verfahren nach einem der Ansprüche 1 bis 12, ferner umfassend zumindest eines der folgenden Merkmale:
- wobei in Batch-Modus Schritt C bei einer Temperatur von etwa 20 °C und 30 °C, insbesondere von etwa 20 °C und 25 °C durchgeführt wird;
- wobei in kontinuierlichem Flussmodus Schritt C bei einer Temperatur in dem Bereich von 20 °C bis 100 °C, bevorzugt um 20 °C durchgeführt wird; und
- wobei in kontinuierlichem Flussmodus in Schritt C die Flussraten eingestellt werden, was in dem Bereich von 0,5 min bis 30 min Verweilzeit, bevorzugt um 10 min, resultiert.

14. Verfahren zur Herstellung von Vilanterol-Trifenatat (Verbindung I) nach einem vorhergehenden Anspruch, wobei das Verfahren Reagieren von Verbindung VI mit Triphenylessigsäure umfasst, um Vilanterol-Trifenatat (Verbindung I) zu bilden.

15. Verfahren zur Herstellung von Vilanterol-Trifenatat (Verbindung I) nach einem der Ansprüche 1 oder 4 oder 5 bis 14, wobei das Verfahren Folgendes umfasst:
i) Entfernen der Acetonid-Schutzgruppe aus Verbindung V in einer sauren Lösung, um Verbindung VI zu bilden; und
ii) Reagieren von Verbindung VI mit Triphenylessigsäure, um Verbindung I zu bilden.

## Revendications

1. Processus permettant la préparation du vilantérol ((R)-4-(2-((6-(2-((2,6-dichlorobenzyl)oxy)éthoxy)hexyl)amino)-1-hydroxyéthyl)-2-(hydroxyméthyl)phénol ; Composé VI) ou d'un sel pharmaceutiquement acceptable de celui-ci, lequel processus comprend les étapes suivantes, les étapes A à B (i) étant réalisées comme un processus réalisé dans un seul récipient :
A) couplage du composé (5R)-5-(2,2-diméthyl-4H-1,3-benzodioxin-6-yl)-1,3-oxazolidin-2-one (Composé II) avec le 2-[2-(6-bromohexyloxy)-éthoxyméthyl]-1,3-dichlorobenzène (Composé III) en présence d'une base, dans un solvant organique ; et
B)
(i) ajout d'une base au mélange réactionnel formé à l'étape A en présence dudit solvant organique pour éliminer le groupe protecteur oxazolidinone, afin de former le Composé V ;
(ii) conversion du Composé V en vilantérol (Composé VI) ou en sel pharmaceutiquement acceptable de celui-ci.

2. Processus selon la revendication 1 permettant la préparation du vilantérol ((R)-4-(2-((6-(2-((2,6- dichlorobenzyl)oxy)éthoxy)hexyl)amino)-1-hydroxyéthyl)-2-(hydroxyméthyl)phénol ; Composé VI) ou d'un sel pharmaceutiquement acceptable de celui-ci, lequel processus comprend les étapes suivantes, les étapes A à C étant réalisées comme un processus réalisé dans un seul récipient :
A) couplage du composé (5R)-5-(2,2-diméthyl-4H-1,3-benzodioxin-6-yl)-1,3-oxazolidin-2-one (Composé II) avec le 2-[2-(6-bromohexyloxy)-éthoxyméthyl]-1,3-dichlorobenzène (Composé III) en présence d'une base, dans un solvant organique ;
B) ajout d'une base au mélange réactionnel formé à l'étape A en présence dudit solvant organique pour éliminer le groupe protecteur oxazolidinone ; et
C) ajout d'une solution acide au mélange réactionnel formé à l'étape B en présence dudit solvant organique pour cliver le groupe protecteur acétonide, afin de former le vilantérol (Composé VI) ;
D) éventuellement, conversion du Composé VI en sel pharmaceutiquement acceptable de celui-ci.

3. Processus selon l'une des revendications 1 ou 2 permettant la préparation d'un sel pharmaceutiquement acceptable de vilantérol, lequel processus comprend les étapes suivantes, les étapes A à D étant réalisées comme un processus réalisé dans un seul récipient :
A) couplage du composé (5R)-5-(2,2-diméthyl-4H-1,3-benzodioxin-6-yl)-1,3-oxazolidin-2-one (Composé II) avec le 2-[2-(6-bromohexyloxy)-éthoxyméthyl]-1,3-dichlorobenzène (Composé III) en présence d'une base, dans un solvant organique ;
B) ajout d'une base au mélange réactionnel formé à l'étape A en présence dudit solvant organique pour éliminer le groupe protecteur oxazolidinone ; et
C) ajout d'une solution acide au mélange réactionnel formé à l'étape B en présence dudit solvant organique pour cliver le groupe protecteur acétonide, afin de former le vilantérol (Composé VI) ;
D) conversion du Composé VI en sel pharmaceutiquement acceptable de celui-ci.

4. Processus selon une quelconque revendication précédente, comprenant en outre au moins l'une des caractéristiques suivantes :
- dans lequel il n'y a pas d'isolement des intermédiaires jusqu'à ce que le Composé V soit obtenu ;
- dans lequel il n'y a pas d'isolement des intermédiaires jusqu'à ce que le Composé VI soit obtenu ; et
- dans lequel il n'y a pas d'isolement des intermédiaires jusqu'à ce qu'un sel pharmaceutiquement acceptable du vilantérol (Composé VI) soit obtenu à l'étape D.

5. Processus selon une quelconque revendication précédente, ledit processus étant réalisé en mode discontinu ou en mode d'écoulement continu.

6. Processus selon une quelconque revendication précédente, comprenant en outre au moins l'une des caractéristiques suivantes :
- dans lequel le solvant organique de l'étape A est choisi dans le groupe constitué de solvants aprotiques polaires, tels que le diméthylformamide, le diméthylsulfoxyde, le diméthylacétamide, la n-méthylpyrrolidin-2-one, d'éthers tels que le tétrahydrofurane, le sulfolane ou de mélanges de ceux-ci ; de préférence le DMSO ;
- dans lequel le volume de solvant par rapport au Composé II est d'au moins 10 volumes, de préférence 25 volumes ;
- dans lequel la base utilisée à l'étape A est choisie dans le groupe constitué d'hydroxydes de métaux alcalins, d'hydrures de métaux alcalins ou d'alcoxydes de métaux alcalins, de préférence le tert-butoxyde de potassium ; et
- dans lequel, en mode discontinu, l'étape A est réalisée à une température de 20 à 30 °C, de préférence de 20 à 25 °C.

7. Processus selon une quelconque revendication précédente, dans lequel, en mode discontinu, la base est utilisée en une quantité molaire au moins stœchiométrique par rapport au Composé II, de préférence d'environ 1 à environ 1,15 équivalents molaires de base, par rapport au Composé II.

8. Processus selon l'une quelconque des revendications 1 à 6, dans lequel, en mode d'écoulement continu, la base est utilisée en une quantité molaire au moins stœchiométrique par rapport au Composé II, de préférence d'environ 1,15 à 3 équivalents molaires de base sont utilisés par rapport au Composé II.

9. Processus selon l'une quelconque des revendications 1 à 6 ou 8, comprenant en outre au moins l'une des caractéristiques suivantes :
- dans lequel, en mode d'écoulement continu, la réaction du Composé II avec une base à l'étape A est réalisée à une température comprise dans la plage de 20 °C à 100 °C, de préférence d'environ 20 °C, et les débits sont ajustés, ce qui donne un temps de séjour compris dans la plage de 0,5 min à 30 min, de préférence d'environ 6 min ; et
- dans lequel, en mode d'écoulement continu, la réaction du Composé II déprotoné avec le Composé III de l'étape A est réalisée à une température comprise dans la plage de 20 °C à 100 °C, de préférence d'environ 60 °C, et les débits sont ajustés, ce qui donne un temps de séjour compris dans la plage de 0,5 min à 30 min, de préférence d'environ 6 min.

10. Processus selon l'une quelconque des revendications 1 à 7, comprenant en outre au moins l'une des caractéristiques suivantes :
- dans lequel, en mode discontinu, la base utilisée à l'étape B est utilisée en une quantité de 1 à 3 équivalents molaires par rapport au Composé II, de préférence 1,5 équivalents molaires par rapport au Composé II, de préférence la base est de l'eau ; et
- dans lequel en mode discontinu, la réaction de l'étape B est réalisée à une température de 30 à 65 °C, de préférence de 35 à 50 °C.

11. Processus selon l'une quelconque des revendications 1 à 6, 8 et 9, comprenant en outre au moins l'une des caractéristiques suivantes :
- dans lequel, en mode d'écoulement continu, la base utilisée à l'étape B est utilisée en une quantité allant jusqu'à 15 équivalents molaires par rapport au Composé II, de préférence d'environ 4 à 5 équivalents molaires par rapport au Composé II ;
- dans lequel, en mode d'écoulement continu, la réaction de l'étape B est réalisée à une température comprise dans la plage de 20 °C à 150 °C, de préférence d'environ 80 °C ; et
- dans lequel, en mode d'écoulement continu à l'étape B, les débits sont ajustés, ce qui donne un temps de séjour compris dans la plage de 0,5 min à 30 min, de préférence d'environ 4 min.

12. Processus selon l'une quelconque des revendications 2 à 11, dans lequel la solution acide utilisée à l'étape C est choisie dans le groupe constitué de solutions de HCl 0,1 à 1N ; de solutions alcooliques de HCl 1 à 10 % ; de HCl dissous dans des solvants organiques appropriés tels que DMF-HCl, éther-HCl, acide acétique-HCl ; de préférence une solution aqueuse de HCl 1N, ou toute solution acide appropriée pour réaliser l'hydrolyse de l'acétal à l'étape C.

13. Processus selon l'une quelconque des revendications 1 à 12, comprenant en outre au moins l'une des caractéristiques suivantes :
- dans lequel, en mode discontinu, l'étape C est réalisée à une température d'environ 20 °C à 30 °C, en particulier d'environ 20 °C à 25 °C ;
- dans lequel, en mode d'écoulement continu, l'étape C est réalisée à une température comprise dans la plage de 20 °C à 100 °C, de préférence d'environ 20 °C ; et
- dans lequel, en mode d'écoulement continu à l'étape C, les débits sont ajustés, ce qui donne un temps de séjour compris dans la plage de 0,5 min à 30 min, de préférence d'environ 10 min.

14. Processus permettant la préparation de trifénatate de vilantérol (Composé I) selon une quelconque revendication précédente, lequel processus comprend la mise en réaction du Composé VI avec l'acide triphénylacétique pour former le trifénatate de vilantérol (Composé I).

15. Processus permettant la préparation de trifénatate de vilantérol (Composé I) selon l'une quelconque des revendications 1, ou 4, ou 5 à 14, lequel processus comprend :
i) l'élimination du groupe protecteur acétonide du Composé V dans une solution acide, pour former le Composé VI ; et
ii) la mise en réaction du Composé VI avec l'acide triphénylacétique pour former le Composé I.
